# EUROPÄISCHE PATENTSCHRIFT

(11) **EP 1 562 534 B1**
(45) Veröffentlichungstag und Bekanntmachung des Hinweises auf die Patenterteilung: **22.09.2010**
(21) Anmeldenummer: 03778315.6
(22) Anmeldetag: 11.11.2003
(51) Int. Cl.: A61Q 1/10, A61K 8/06, A61K 8/34, A61K 8/73, A61K 8/04

(54) **KOSMETISCHE ZUBEREITUNG MIT WACHSKOMPONENTE, FILMBILDNER UND GELKOMPONENTE**
COSMETIC DRESSINGS CONTAINING A WAXY COMPONENT, A FILM-FORMING AGENT AND A GEL-FORMING SUBSTANCE
PREPARATION COSMETIQUE CONTENANT UN CONSTITUANT CIREUX, UN AGENT FILMOGENE ET UN CONSTITUANT GELIFIANT

(30) Priorität: 13.11.2002 DE 10252816
(43) Veröffentlichungstag der Anmeldung: 17.08.2005
(73) Patentinhaber: Schwan-STABILO Cosmetics GmbH & Co. KG, 90562 Heroldsberg (DE)
(72) Erfinder: PÄTZER, Maike, 91086 Aurachtal (DE); SWISTOWSKI, Azra, 90491 Nürnberg (DE); ZECH, Christina, 86916 Kaufering (DE)
(74) Vertreter: Leissler-Gerstl, Gabriele
(86) Internationale Anmeldenummer: PCT/EP2003/012595
(87) Internationale Veröffentlichungsnummer: WO 2004/043410

(56) Entgegenhaltungen:
- EP-A- 1 352 639
- EP-A- 1 352 641
- WO-A-01/01949
- WO-A-94/17780
- DE-A- 3 321 650
- DE-U- 29 919 474
- US-A- 5 800 818
- US-B1- 6 325 994

## Beschreibung

Die Erfindung betrifft eine kosmetische Zubereitung, insbesondere eine Zubereitung zum Färben und Formen von keratinischern Material, sowie ein Verfahren zu ihrer Herstellung und ihre Verwendung.

Kosmetische Zubereitungen, mit denen keratinisches Material, z.B. Wimpern, gefärbt werden kann, sind schon seit langer Zeit bekannt Um den vielfältigen Anforderungen, die an eine derartige Zubereitung zu stellen sind, gerecht zu werden, wurden immer wieder Verbesserungen entwickelt. Zusammensetzungen, die auf Wimpern aufgetragen werden, sollen einerseits die Wimpern gut färben, nicht jedoch auf die Umgebung abfärben. Sie sollen an den Wimpern gut haften, sich jedoch auch leicht wieder entfernen lassen. Sie sollen sich leicht auftragen lassen, den Wimpern eine schöne Form geben und diese Form auch nach dem Trocknen behalten. Weiterhin sollen die Zusammensetzungen so dünnflüssig sein, dass sie sich gut auftragen lassen, andererseits aber schnell trocknen, damit die Masse nicht verschmiert wird. Darüberhinaus sollen die Produkte so stabil sein, dass sie längere Zeit unter Umgebungsbedingungen aufbewahrt werden können.

Zur Lösung all dieser, sich teilweise widersprechenden Aufgaben wurden vielfältige Materialien vorgeschlagen. Gängige Mascaras enthalten einen Wachsanteil, der für die Haftung auf der Wimper sorgt, einen Filmbildner, der für eine schöne Form und das Erhalten dieser Form sorgt sowie Bindemittel, um die Viskosität in einem geeigneten Bereich einzustellen. Häufig sind Mascaras Emulsionen, bei denen die kontinuierliche Phase von Wasser gebildet wird, während die diskontinuierliche Phase von der Wachskomponente gebildet wird. So beschreibt beispielsweise US-A 6.210.692 kosmetische Emulsionen, die u.a. als Mascara geeignet sind und aus einer wässrigen Phase und einer Fettphase aufgebaut sind, die zur Stabilisierung zwei verschiedene Bindemittel, nämlich ein hydrophiles Verdickungsmittel und einen Polysaccharidether enthalten, wobei die Verwendung solcher Verdickungsmittel, die Gele mit der wässrigen Phase bilden, vermieden werden soll. Diese Emulsionen sollen besonders gut geeignet sein, um trockene und empfindliche Haut zu pflegen und zu behandeln.

Aus DE 33 21 650 A sind kosmetische Zubereitungen bekannt, die mindestens ein Wachs mit einem Schmelzpunkt zwischen 60 und 110 °C, sowie mindestens ein kationisches und ein anionisches Polymer aufweisen. Die Zubereitungen zeichnen sich durch eine verbesserte Stabilität und gleichmäßigere Farbverteilung aus.

Weiterhin ist aus US-A 6.325.994 eine kosmetische Zusammensetzung bekannt, die insbesondere als Mascara geeignet ist, die aus einer lipophilen Phase mit Fett und Wachs, sowie einem lipophilen Polymer besteht, wobei das lipophile Polymer ein spezielles Acrylat-Methacrylat-Copolymer ist. Die Zusammensetzung kann auch in Form einer Emulsion mit einer wässrigen Phase vorliegen. Die Zusammensetzung kann darüber hinaus übliche Zusatzstoffe wie Farbstoffe, Pigmente und Füllstoffe enthalten. Obwohl diese Zusammensetzung wasserbeständig und sehr haltbar sein soll, befriedigt sie noch nicht in jeder Hinsicht.

In der Literatur werden viele weitere ähnliche Zusammensetzungen beschrieben, die üblicherweise Wachse, Öle, Bindemittel und Färbemittel enthalten und auch in Form von Öl-in-Wasser-Emulsionen vorliegen können. Die bekannten Zusammensetzungen weisen jedoch mindestens einen der folgenden Nachteile auf. Viele der bekannten Emulsionen sind nicht stabil genug, um in erwärmtem Zustand verarbeitet zu werden. Da jedoch für kosmetische Zusammensetzungen eine Wärmebehandlung notwendig ist, erfordert dies spezielle Behandlungsstufen, die aufwändig sind.

Aufgrund ihrer Zusammensetzung ist es häufig schwierig, Pigmente so einzuarbeiten, dass sie stabil .in der Zusammensetzung bleiben ohne sich abzusetzen oder zu verklumpen. Zusammensetzungen, die gut auf keratinischern Material haften, sind oft schwierig wieder davon zu entfernen. Insbesondere wasserfeste Zusammensetzungen lassen sich durch wässrige Zubereitungen nicht mehr entfernen. Ein großes Problem, das insbesondere bei Mascaras auftritt, besteht darin, dass Teilchen aus der Zusammensetzung auswandern und sich entweder auf der Haut absetzen, was unschöne Verfärbungen ergibt, oder, was noch gravierender ist, in das Auge gelangen und z.B. bei Linsenträgerinnen die Linsen verfärben und damit unbrauchbar machen können.

Die Erfinder der vorliegenden Anmeldung haben sich daher als Aufgaben gestellt, ein Produkt zu entwickeln, das in der Herstellung einfach ist und ohne besonderen Maschinenpark sicher und reproduzierbar hergestellt werden kann; in das Pigmente leicht eingearbeitet werden können und stabil darin enthalten bleiben; das auch bei höherer Temperatur stabil ist und ohne Trennung oder Zersetzung erwärmt werden kann; das bei erhöhter Temperatur eine solche Viskosität aufweist, dass es leicht abfüllbar ist; das leicht aufgetragen werden kann, lange haftet, die Wimpern verlängern kann, den Wimpern Volumen geben kann, sich nicht auf Lidflächen überträgt, wasser- und tränenfest ist, nach dem Antrocknen nicht abbröselt und leicht wieder entfernt werden kann.

All diese Aufgaben werden erfindungsgemäß gelöst mit einer kosmetischen Zubereitung, die in Form einer O/W-Emulsion vorliegt und neben üblichen kosmetischen Inhaltsstoffen eine Wachskomponente, einen Filmbildner und mindestens eine Gelkomponente aufweist, wobei die Gelkomponente mindestens ein gequollenes Hydrokolloid enthält, wobei die kosmetische Zubereitung ein wässriges Gel und ein alkoholisches Gel enthält, wobei das alkoholische Gel ein mit einem einwertigen und/oder mehrwertigen Alkohol gequollenes Cellulosederivat ist, wobei der Alkohol insbesondere ein C₂-C₄ Alkohol ist under mehrwertige Alkohol insbesondere ein C₂-C₆ Alkohol ist.

Es wurde überraschenderweise gefunden, dass der Einsatz eines gequollenen Hydrokolloids der Zubereitung Struktur geben kann und außerdem Färbemittel und Pigmente so binden kann, dass diese nicht mehr aus der Masse herauswandern können, so dass die aufgetragene getrocknete Masse selbst bei Kontakt mit Wasser nicht abfärbt. Darüber hinaus wird durch das gequollene Hydrokolloid die Struktur der Zubereitung in festem und flüssigem Zustand so stabilisiert, dass selbst bei höherer Temperatur keine Entmischung auftritt. Dies hat den Vorteil, dass sie problemlos erwärmt und in erwärmtem Zustand abgefüllt werden kann. Die Herstellung der erfindungsgemäßen Zubereitung ist einfach, und kann mit üblichen, zur Verfügung stehenden Apparaturen leicht und sicher erfolgen.

Die kosmetische Zubereitung ist insbesondere dafür vorgesehen, auf keratinisches Material aufgetragen zu werden, wobei vor allem Augenwimpern, Augenbrauen, Haare und Haarteile in Betracht gezogen werden. Besonders geeignet ist die erfindungsgemäße Zubereitung als Mascara.

Die wichtigsten Bestandteile der erfindungsgemäßen Zubereitung sind neben dem die kontinuierliche Phase der Emulsion bildenden Wasser eine Wachskomponente, ein Filmbildnersystem und mindestens eine Gelkomponente.

Die Wachskomponente besteht aus mindestens einem Wachs und bevorzugt zusätzlich mindestens einem Öl und/oder Fett, die jeweils pflanzlicher, tierischer, mineralischer oder synthetischer Herkunft sein können. Darüber hinaus kann vorzugsweise mindestens ein Emulgator und ggf. zusätzlich mindestens ein Coemulgator enthalten, sein, um die Verarbeitung der Wachskomponente zu einer Emulsion zu erleichtern. Um ein ästhetisch besonders befriedigendes Endprodukt zu erhalten kann die Wachskomponente zusätzlich ein Vinylpyrrolidon-Copolymer enthalten.

Die Wachskomponente gibt der Masse Konsistenz und macht die Zusammensetzung wasserfest. Zu diesem Zweck kann die Wachskomponente aus fett-, öl- und wachsartigen Substanzen aufgebaut sein, die bei Raumtemperatur flüssig, pastenförmig oder fest sein können. Bevorzugt wird zur Einstellung der optimalen Konsistenz eine Kombination aus mindestens einem Wachs und mindestens einem Öl eingesetzt.

Bevorzugt wird mindestens ein Wachs verwendet, das bei Raumtemperatur fest ist. Besonders bevorzugt sind solche Wachse mit einem Tropfpunkt von 50 bis 200°C. Wachse mit einem Tropfpunkt unter 50°C können Probleme bei der Lagerfähigkeit schaffen und Wachse mit einem Tropfpunkt über 200°C sind teilweise in der Verarbeitung schwierig.

Für die erfindungsgemäß eingesetzte Wachskomponente sind die üblicherweise in Kosmetika verwendeten Wachse geeignet, insbesondere pflanzliche Wachse, wie Carnauba und- Candelillawachse, Ouricurriwachs, Japanwachse, Baumwollwachs, Reiswachs, Blütenwachse, hydriertes Jojobaöl; tierische Wachse, wie Bienenwachse und modifizierte Bienenwachse, u.a. Cera Bellina, Lanolinwachse und Insektenwachse; mineralische Wachse, wie Paraffinwachse, mikrokristalline Wachse, Montanwachse und Ozokerite, sowie synthetische Wachse, wie Fischer-Tropsch-Wachse, Wachs-Polymer-Hybride, Polyethylenwachse, Siliconwachse und Acelainsäuredioleylester, Acelainsäuredibehenylester, Behenyloleat, Cetylpalmitat und Mischungen aller aufgeführten Wachse. Besonders bevorzugt werden tierische Wachse und synthetische Wachse, sowie insbesondere deren Gemische eingesetzt.

Mindestens ein Fett oder Öl ist in der Wachskomponente enthalten, um die Viskosität der Masse einzustellen und der Masse Geschmeidigkeit zu verleihen. Hierzu kommen sowohl tierische als auch pflanzliche Fette und Öle in Betracht, die auch in hydriertem oder modifizierten Zustand eingesetzt werden können. Beispiele für geeignete Fette und Öle sind Rapsöl, Sonnenblumenöl, Sesamöl, Erdnussöl, Distelöl, Cocosöl, hydriertes Cocosöl, Ricinusöl, hydriertes Ricinusöl, Rindertalg, Isopropylmyristat, Isopropylpalmitat, Isopropylstearat, Isobutylstearat, Isostearylisostearat, Oleyloleat, Jojobaöl, Caprylic/CapricTriglyceride und ähnliche synthetische Trygliceride, Paraffinöl, Polybuten, Squalan, Sqalen und deren Gemische, synthetische Ester, Siliconöle, wobei hier sowohl flüchtige als auch nicht-flüchtige Öle in Betracht kommen, insbesondere Dimethicone und Cyclomethicone sowie flüchtige und nicht-flüchtige Isoparaffine. Mischungen dieser Bestandteile sind bevorzugt. So ist häufig sowohl ein nichtflüchtiges Fett oder Öl als auch ein flüchtiges Öl enthalten, um verschiedene Eigenschaften zu vermitteln. In einer Ausführungsform enthält die Wachskomponente flüchtige Bestandteile, insbesondere synthetische Ester, Siliconöle, Dimethicone und Cyclomethicone und Isoparaffine in einer Konzentration von bis zu 20 %.

Wie erwähnt werden bevorzugt Mischungen aus Wachsen, Fetten und Ölen verwendet, wobei die jeweils ausgewählten Substanzen in solchen Mengen eingesetzt werden, dass die gewünschten Eigenschaften, wie Textur und Viskosität, erzielt werden. Die jeweils einzusetzenden Mengen und Mischungen sind dem Fachmann bekannt und bedürfen keiner näheren Erläuterung.

Die Wachskomponente kann darüber hinaus noch weitere dem Fachmann an sich bekannten Bestandteile aufweisen, die Eigenschaften wie Stabilität, Viskosität, Verarbeitbarkeit und Haltbarkeit beeinflussen. So können z.B. Zuckerderivate enthalten sein.

Bevorzugt wird die Wachskomponente in einem solchen Anteil eingesetzt, dass sie 0 bis 50 %, bevorzugt 15 bis 30 % der fertigen Zusammensetzung bildet, wobei sich die Prozentangabe, wie alle Angaben in der Beschreibung, falls nicht anders angegebenen, auf Gewicht bezieht. Innerhalb der Wachskomponente liegt bevorzugt der Anteil des Wachses bzw. der Wachse zwischen 20 und 100 % und ist besonders bevorzugt zwischen 70 und 95 %.

Das Fett oder Öl wird üblicherweise in einem solchen Anteil zugegeben, dass die Konsistenz der Masse im gewünschten Bereich ist. Normalerweise liegt der Anteil der Fette und Öle in der Wachskomponente, abhängig von den eingesetzten Materialien, zwischen 20 und 80 %, wobei der untere Bereich bevorzugt ist.

Zusätzlich kann die Wachskomponente, wie oben ausgeführt, noch Emulgatoren und ggf. zusätzlich Coemulgatoren enthalten. Diese fördern die Emulsionsbildung und Homogenität und stabilisieren die gebildete Emulsion. Die Auswahl dieser Bestandteile ist nicht kritisch, sie müssen jedoch die für kosmetische Zusammensetzungen geforderten Eigenschaften aufweisen, dürfen insbesondere nicht reizend und nicht toxisch sein. Alle üblicherweise in Kosmetika verwendeten und dem Fachmann bekannten können hier eingesetzt werden. Beispiele sind anionaktive, kationaktive, nichtionogene Emulgatoren und Amphotenside, sowie Polymertenside, die z.B aus Copolymeren, Blockcopolymeren und Pfropfpolymeren aufgebaut sein können, Silicontenside und polymere Silicontenside, Siliconcopolymere, PEG-Derivate natürlicher Fettsäuren und Betaine.

Die Emulgatoren und Coemulgatoren werden der Wachskomponente in den üblichen Mengen zugegeben; falls vorhanden, liegt die bevorzugte Menge (bezogen auf die Wachskomponente) zwischen etwa 0,5 und 40 %. Die optimale Menge hängt unter anderem ab von Art und Menge der Wachskomponente und der Art der eingesetzten Emulgatoren und kann leicht durch Routineversuche festgestellt werden. Besonders gute Ergebnisse werden erhalten bei einem Anteil im Bereich von 10 bis 25 %.

Die zweite wesentliche Komponente der erfindungsgemäßen kosmetischen Zubereitung ist ein Filmbildnersystem. Das Filmbildnersystem wird üblicherweise von einem Polymer oder mehreren Polymeren, das/die in einem Medium in Dispersion oder gelöst vorliegen, gebildet, wobei das/die Polymer(en) bei der Entfernung des Mediums, d.h. des Lösungs- bzw. Dispersionsmittels, einen Film bilden. Der Feststoffgehalt liegt je nach Polymerart und Medium üblicherweise zwischen 10 und 70 %, bevorzuugt - zwischen 20 und 40 %. In der vorliegenden Anmeldung wird jedes System aus Filmbildner und Medium (auch Solubilisierungsmittel, Lösungsmittel oder Dispersionsmittel genannt) generell als Dispersion bezeichnet, um Unklarheiten auszuschließen, da auf dem Gebiet der Polymere die Übergänge zwischen Lösung und Dispersion fließend sind. Der Ausdruck "Dispersion" schließt daher auch Lösungen mit ein. Eingeschlossen sind auch Systeme, in denen das filmbildende Polymer z.B. durch Zugabe einer Säure oder Base solubilisiert wurde. Das Medium ist bevorzugt Wasser.

Filmbildende Polymere sind an sich bekannt und alle üblicherweise für kosmetische Präparate verwendeten können zum Einsatz kommen. Der Filmbildner führt zusammen mit der Wachskomponente zu einem sehr haltbaren Überzug für das keratinische Material, z.B für Wimper oder Augenbraue oder Haar.

Das filmbildende Polymer kann ein Polykondensat, radikalisch erzeugtes Polymer oder ein Polymer natürlichen Ursprungs sein. Filmbildnersysteme, die für die erfindungsgemäße Zubereitung besonders geeignet sind, enthalten anionische, kationische, nichtionische und/oder amphotere Polymere aus der Klasse der Polyurethane, der Polyharnstoffe, Polyester und Polyether sowie deren Derivate, PVP, Polyamide, Vinyl-, Acryl- und Methacrylpolymere und - copolymere, Epoxyester, Siliconpolymere und aus den oben erwähnten Polymeren gebildete Copolymere und Hybridpolymere. Das Filmbildnersystem liegt üblicherweise als Dispersion vor, wobei wässrige Systeme bevorzugt werden.

Besonders bevorzugte Filmbildner sind Polyurethane und PolyurethanCopolymere, wie Polyurethan, z.B. Polyurethan-Acrylsäure-Copolymere, Polyurethan-Polyvinylpyrrolidone, Polyesterpolyurethane, Polyetherpolyurethane, Polyharnstoffe, Polyesteramide, Polyester mit Fettkette, Polyamide, Epoxyesterharze und/oder Polypropylenglykol-Maleinester-Copolymere. Viele dieser Polymere sind im Handel erhältlich. Eine weitere bevorzugte Gruppe sind Acrylpolymere, Acryl/Styrolcopolymere, Acryl/Vinylcopolymere und Acryl/Siliconcopolymere ebenso wie Kombinationen aus Nitrocellulose und Acrylpolymer. Auch Hybridpolymere können erfindungsgemäß eingesetzt werden. Polymere natürlichen Ursprung, die gegebenenfalls auch in modifizierter Form vorliegen können, wie beispielweise Polysaccharid- und Cellulosederivate, sind geeignet. Eine Mischung von zwei oder mehr der aufgezählten Polymere kann ebenfalls eingesetzt werden, wobei die Mischung so gebildet wird, dass die gewünschten Eigenschaften erzielt werden. Besonders geeignet sind auch die von der CTFA zugelassenen Produkte, insbesondere Polyurethan-1 bis Polyurethan-13.

Das Filmbildnersystem wird in einer Menge verwendet, die den gewünschten Effekt liefert. Geeignet sind Mengen im Bereich von etwa 5 bis 95 %. Um besonders vorteilhafte Eigenschaften, z.B. inbezug auf die Haltbarkeit und Haftung zu erhalten, werden Mengen im Bereich von 5 bis 60 %, bevorzugt 20 bis 35 % eingesetzt, wobei die Menge im Einzelfall von dem verwendeten Polymer und Anteil und Art der Wachskomponente und Gelkomponente abhängt. Je stärker die von der Gelkomponente gebildete Struktur bzw. je stärker der gebildete Film ist, desto geringer kann die Menge an Filmbildnersystem sein. Die optimale Menge kann vom Fachmann leicht gefunden werden. Die Mengenangabe bezieht sich dabei auf das verwendete System, d.h. die Menge an filmbildenden Polymeren und Solubilisierungsmittel.

Die erfindungswesentliche Komponente der kosmetischen Zubereitung ist eine Gelkomponente, die enthalten sein muss, um die vorteilhaften Eigenschaften zu erzielen. Es wurde festgestellt, dass die Verwendung eines gequollenen Hydrokolloids als Gelkomponente strukturbildend wirkt und die Struktur so stabilisiert, dass die Zusammensetzung nach dem Trocknen einen dauerhaften, wasserfesten Film auf keratinischem Material bildet und zusammen mit dem Filmbildner und der Wachskomponente alle Inhaltsstoffe so in der Zusammensetzung festhält, dass sie nicht ausgelaugt werden, auswandern oder ausbluten. Durch die Gelkomponente wird daher ein Material geschaffen, das ideal geeignet ist, um Färbemittel und Pigmente und auch weitere Inhaltsstoffe aufzunehmen und festzuhalten.

Die Gelkomponente wird aus mindestens einem gequollenem Hydrokolloid gebildet, wobei die Quellung bevorzugt mit Wasser erfolgt. Es wurde festgestellt, dass ein Hydrokolloid in gequollenem Zustand das gewünschte Netzwerk aufspannt, das dann in Kombination mit dem Filmbildnersystem andere Inhaltsstoffe aufnehmen und stabilisieren kann. Als Hydrokolloide sollen im Zusammenhang mit der vorliegenden Erfindung solche natürlichen und synthetischen Polymere bezeichnet werden, die in Lösungsmitteln, insbesondere wässrigen Systemen Gele oder viskose, insbesondere hochviskose Lösungen bilden. Beispiele sind insbesondere Cellulosen und Cellulosederivate, Stärke und Stärkederivate, Alginate, Carageene, Pektine, Tragant und Gummen sowie Polyvinylalkohol und Polyvinylpyrrolidon und auch Dextran. Obwohl diese Stoffe in Pulverform teilweise bereits zur Verwendung als Verdickungsmittel in Kosmetika bekannt sind, wurde bisher nicht erkannt, dass sie in vorgequollenem Zustand einer kosmetischen Zubereitung besonders vorteilhafte Eigenschaften vermitteln können. Es wurde nun überraschenderweise gefunden, dass diese Hydrokolloide in gequollenem Zustand der erfindungsgemäßen Zubereitung Struktur, Stabilität und Volumen verleihen. Die Verwendung des erfindungsgemäßen gequollenen Hydrokolloids kann sogar dazu führen, dass sich das Volumen der Zubereitung nach dem Trocknen kaum oder nicht verringert. Dadurch macht die erfindungsgemäße Zubereitung auch nach dem Trocknen die Wimpern füllig und länger und verstärkt den ästhetischen Eindruck.

Wesentlich ist daher, das Hydrokolloid in ausgequollenem Zustand für die Herstellung der erfindungsgemäßen Zubereitung zu verwenden und es nicht erst in der Zubereitung quellen zu lassen. Besonders gute Eigenschaften werden erzielt, wenn das Hydrokolloid in erwärmtem Wasser gequollen wird, insbesondere in Wasser mit einer Temperatur von 40 bis 100 C°. Üblicherweise wird das Hydrokolloid mit dem Wasser vermischt, z.B. unter Rühren, und dann bevorzugt vor der Weiterverarbeitung mindestens auf ca. 40°C, besonders bevorzugt auf Raumtermperatur abkühlen gelassen. Durch das Quellen verändern sich die physikalischen Eigenschaften des Hydrokolloids irreversibel, was die gewünschte Struktur liefert. Das so erhaltene vorgequollene Hydrokolloid ist stabil und lagerfähig und kann daher vor der Weiterverarbeitung auch einige Zeit, z.B. einige Stunden, oder auch noch längere Zeiträume aufbewahrt werden.

Es wird angenommen, dass sich durch das Quellen die Wasserbindekapazität erhöht, so dass die fertige Zusammensetzung später dann mehr Wasser binden bzw. halten kann, was unter anderem zu den vorteilhaften Eigenschaften führt.

Bevorzugt wird daher ein Hydrokolloid mit einer Wasserbindungskapazität von 100 bis 600 %, bevorzugt 200 bis 500 % und insbesondere 350 bis 430 % verwendet. Die Wasserbindungskapazität des Hydrokolloids kann mit an sich bekannten Methoden bestimmt werden, z.B. mit Kart-Fischer-Titration oder Trockenrückstandsbestimmung.

Weiterhin wurde gefunden, dass ein Hydrokolloid mit einem hohen Quellungsvermögen bei 80°C besonders vorteilhaft ist. Daher sind Hydrokolloide bevorzugt, deren Quellungsvermögen in einem Bereich von 250 bis 500 %, gemessen bei 80°C, liegt Das Quellungsvermögen kann in an sich bekannter Weise bestimmt werden, z.B. indem das Hydrokolloid in Wasser erhitzt wird und nach homogenem Aufquellen (d.h. ohne Klumpenbildung) unter Rühren auf Raumtemperatur abgekühlt wird, der Wasserüberstand dekantiert wird und das aufgenommene Wasser mit der Methode nach Karl Fischer oder auf einer Trockenwaage bestimmt wird.

Ein besonders vorteilhaftes Quellungsvermögen und eine besonders hohe Wasserbindungskapazität wurden bei modifizierter Stärke festgestellt, die daher bevorzugt eingesetzt wird. Beispiele für geeignete Stärken sind pflanzliche Stärken, insbesondere Getreidestärken, wie Weizen-, Mais-, und Reisstärken, wobei Reisstärke in modifizierter Form besonders bevorzugt ist.

In einer bevorzugten Ausführungsform wird als Hydrokolloid Stärke oder ein Stärkederivat bzw. derivatisierte oder modifizierte Stärke, insbesondere Reisstärke eingesetzt. Besonders vorteilhafte Eigenschaften können mit Hydroxyalkyl- oder Dimethylimidazolidinonreisstärke erhalten werden.

Das ausgequollene Hydrokolloid kann aufgrund seiner Stabilität längere Zeit aufbewahrt werden, bevor es zur Herstellung der kosmetischen Zubereitung weiterverarbeitet wird. Um zu verhindern, däss das gequollene Hydrokolloid bakteriell verseucht wird, kann dem System aus Hydrokolloid und Wasser ein Konservierungsmittel zugefügt werden. Konservierungsmittel für derartige Systeme sind dem Fachmann wohlbekannt und die für Lebensmittel und Kosmetika geeigneten können hier eingesetzt werden.

Zusätzlich kann dem. System auch noch ein Dispergierhilfsmittel zugefügt werden. Es wurde gefunden, dass eine Kombination aus Konservierungsmittel und Dispergierhilfsmittel die keimabtötende Wirkung synergistisch erhöht. Als geeignete Konservierungsmittel können Benzylalkohol und die Ester davon, Parabene und deren Salze, Phenyl ethylalkohol, IPBC, Fomaldehydabspalter, wie Diimidazolidinyl-Harnstoff, organische aromatische Säuren, Phenoxyethanol und andere genannt werden. Beispiele für Dispergierhilfsmittel sind PEG-Derivate, nichtionische Tenside, Blockpolymere, Ester organischer Mehrfach-Säuren, Seifen mehrwertiger Metallsalze, Aminosäuren bzw. deren Ester, Siliconylacrylatcopolymere und andere bekannte Netzmittel.

Zur Stabilisierung der Zubereitung können weiterhin Antioxidantien, wie Tocopherol, NDGA, Sesamöl, γ-Oryzanol, Rosmarinöl, BHT und sonstige für Kosmetika eingesetzte Mittel verwendet werden. Die mikrobiologische Stabilität kann bei Bedarf durch Zusatz von Konservierungsmitteln, wie sie für Kosmetika üblich sind, sichergestellt werden.

Der Anteil der Gelkomponente (d.h. Hydrokolloid In gequollenem Zustand) in der erfindungsgemäßen Zusammensetzung kann in einem breiten Bereich variieren. Bevorzugt ist ein Anteil von 3 bis 50 %, besonders bevorzugt 3 bis 20 %. Dieser Anteil bezieht sich auf das Gewicht an gequollenem Hydrokolloid, d.h. Hydrokolloid incl. dem aufgenommenen Anteil an Flüssigkeit, bezogen auf die Zusammensetzung. Unter einem Anteil von 3 % ist die Wirkung nicht mehr optimal und bei einem Anteil von mehr als 50 % kann die Viskosität und Struktur der Zusammensetzung beeinträchtigt sein. Der Anteil des Hydrokolloids in der gequollenen Gelkomponente kann abhängig von der Art des Hydrokolloids auch in breiten Grenzen variieren, und kann je nach Quellfähigkeit z.B. 5 bis 90% sein.

Die Gelkomponente der erfindungsgemäßen Zubereitung enthält weiterhin ein zweites Gel eines natürlichen Polymers, nämlich einem Cellülosederivat, in einem ein- und/oder mehrwertigen Alkohol. Das zweite "alkoholische" Gel dient dazu, die Struktur zu stabilisieren und die rheologischen Eigenschaften zu beeinflussen. Darüberhinaus kann es die Trocknungseigenschaften der Zubereitung beeinflussen. Die alkoholische Gelkomponente ist zwingend erforderlich. Das Polymer des zweiten Gels kann mit dem Hydrokolloid des ersten Gels identisch sein, ist aber bevorzugt davon verschieden.

Das zweite Gel enthält ein mindestens Cellulosederivat, der in Wasser und Alkohol oder in Mischungen aus Wasser und Alkohol löslich ist, insbesondere Carboxymethycellulose, Hydroxymethylcellulose, Hydroxyethylcellulose oder Hydroxypropylcellulose, wobei Hydroxypropylcellulose besonders bevorzugt ist. Das Cellulosederivat wird in dem Alkohol so gelöst bzw. dispergiert, dass sich ein Gel bildet. Dazu wird bevorzugt eine Mischung aus einem oder mehreren einwertigen Alkohol(en) mit einer Kettenlänge von C₂-C₄ und einem mehrwertigen Alkohol mit einer Kettenlänge zwischen C₂ und C₆ und mit mindestens zwei Hydroxylgruppen verwendet. Beispiele für geeignete einwertige Alkohole sind Ethanol, n-Propanol, i-Propanol, n-Butanol, i-Butanol, wobei Ethanol bevorzugt ist. Beispiele für mehrwertige Alkohole sind Propandiole, wie 1,2-Propandiol, Dipropandiol, Butandiole, wie 1,2-Butandiol, 1,3-Butandiol, 1,4-Butandiol, Glycerin, Diglycerin, Triglycerin, Diethylenglycole, Amylalkohol, Hexandiole, wie 1,2-Hexandiol, 1,3-Hexandiol, Pentaerythrit, Sorbit, Xylit, Mannit und Aldit. Bevorzugt ist der mehrwertige Alkohol 1,2-Propandiol oder 1,3-Butandiol. Bevorzugt wird das Cellulosederivat zuerst in dem mehrwertigen Alkohol aufgeschlämmt und dann der einwertige Alkohol zugegeben, bis die gewünschte Viskosität erreicht ist. Dabei bildet sich ein sehr dünnflüssiges Gel, das dann zusammen mit den weiteren Komponenten der erfindungsgemäßen Zubereitung vermischt werden kann.

Die Menge des in der erfindungsgemässen Zubereitung enthaltenen alkoholischen Gels richtet sich nach der gewünschten Viskosität, sowie den Trocknungseigenschaften. Eine Menge von 0 bis 10 % (Polymer + Alkohole) hat sich als geeignet erwiesen. Der Anteil des Polymers liegt dabei in einem Bereich von 0,5 bis 20 % des Gels. Wenn die Zubereitung sehr langsam trocknet, kann der Anteil des alkoholischen Gels bzw. der Anteil des Alkohols in dem Gel erhöht werden.

Die erfindungsgemäße Zubereitung kann außerdem noch weitere übliche kosmetische Inhaltsstoffe enthalten, die bestimmte erwünschte Eigenschaften beeinflussen. Ein wichtiger Inhaltsstoff für die meisten kosmetischen Produkte sind Färbemittel oder Pigmente, um eine wünschenswerte Farbe zu erzeugen. Es wurde gefunden, dass die erfindungsgemäße Zubereitung dafür geeignet ist, sowohl organische Färbemittel als auch Pigmente so zu stabilisieren, dass sie nicht ausgelaugt werden. Als Pigmente oder Färbemittel können alle für Kosmetika bekannten Stoffe eingesetzt werden. Voraussetzung ist nur, dass die Stoffe nicht toxisch und reizend sind. Die Pigmente werden bevorzugt in mikronisierter Form eingesetzt. Beispiele für Pigmente sind Eisenoxide, Ultramarin, Chromoxidgrün, Chromoxidhydratgün, Ruß, Titandioxid, Zinkoxid, Bariumsulfat, Talkum und Kaolin. Beispiele für organische Färbemittel sind Lacke wie Aluminium-, Barium-, Calcium-, Kalium-, Strontium- und Zinklacke, Carmin und andere dem Fachmann wohl bekannte färbende Stoffe. Weiterhin können auch Perlglanzpigmente enthalten sein. Die teilchenförmigen Inhaltstoffe werden in dem üblicherweise für Kosmetika verwendeten Anteil eingesetzt, wobei die Menge abhängig von der Art der Teilchenphase und der gewünschten Wirkung ausgewählt wird. Bevorzugt liegt der Anteil der teilchenförmigen Inhaltsstoffe in einem Bereich von bis zu 30 %, besonders bevorzugt 2 bis 20 %.

Außerdem kann die erfindungsgemäße Zubereitung Füllstoffe und teilchenförmige Stoffe, die der Masse eine gewünschte Struktur geben, enthalten. Auch diese Füllstoffe und Teilchen werden von der erfindungsgemäßen Zubereitung stabilisiert, so dass sie sich nicht absetzen. Beispiel sind Talkum, Kaolin, Bentonit, Hectorit, Montmorillonit, Ceroxid, Siliciumdioxid, Bornitrid, Nylonpulver, Polyethylenpulver, Polypropylenpulver, Seidenpulver und deren Gemische, mehrwertige Metallseifen, nichtquellbare Stärken, Fruchtfasern, natürliche und synthetische Peelingkörper, Sand, Kleieprodukte, Kemmehl von Steinfrüchten, Algenderivate, Duroplast-Thermoplast- und Elastomermehle und Mischungen sowie Hybrides der aufgeführten Bestandteile.

Um die Zubereitung samtig und glänzend zu machen, können auch Weichmacher zugesetzt werden. Beispiele hierfür sind mehrwertige Alkohole und deren Ester, wie Glycerin, Diglycerin, Triglycerin, Diethylenglycole, Amylalkohol, Hexandiole, Pentaerythrit, Sorbit, Xylit, Mannit und Aldit. Saccharoseacetatisobutyrat, Laureth-2-benzoat, Ethylhexylsebacat, Citronensäureester, wie Tributylcitrat, synthetische kurzkettige Ester, Pentaerithritylester und Oligopentaerithritylester.

Wenn die erfindungsgemäße Zubereitung als Mascara verwendet wird, werden üblicherweise solche Pigmente und Färbemittel eingesetzt, die zu den üblicherweise gewünschten Farben, nämlich Schwarz, Blau, Braun und Grau führen. Ebenso können jedoch auch andersfarbige Zubereitungen hergestellt werden, um z.B. die Haar- bzw. Wimpernspitzen in anderer Farbe anzufärben, als den Rest des Haars bzw. der Wimper.

Die färbenden Bestandteile, d.h. Pigmente und Färbemittel müssen nicht notwendigerweise in der erfindungsgemäßen Zubereitung enthalten sein. Es ist auch eine Ausführungsform möglich, in der färbende und strukturgebende Bestandteile getrennt sind und zuerst die erfindungsgemäße Zubereitung ohne färbende Inhaltstoffe auf das keratinische Material aufgetragen wird und anschließend eine Formulierung der Färbemittel oder Pigmente aufgebracht wird.

Der pH-Wert der erfindungsgemäßen Zubereitung sollte bevorzugt im neutralen bis schwach basischen Bereich liegen, da viele der üblicherweise in Kosmetika verwendeten Filmbildner bereits bei schwach saurem pH-Wert ausfallen können. Gegebenenfalls enthält die Zubereitung daher zur Einstellung bzw. Abpufferung des pHs noch basische Mittel und/oder Puffermittel, z.B. NaOH, Amine und übliche Puffer. Bevorzugt wird der pH-Wert der Zubereitung so eingestellt, dass er in einem Bereich von 6,5 bis 8,8, besonders bevorzugt 6,8 bis 7,5 liegt:

Erfindungsgemäß wird somit eine Zubereitung zur Verfügung gestellt, die in Form einer Emulsion vorliegt und besonders vorteilhafte Eigenschaften vereinigt. Die Zubereitung kann leicht hergestellt werden, führt zu stabilen Produkten, kann leicht weiterverarbeitet werden, da sie wärmestabil ist und erzeugt einen Film, der lange haftet, ohne die darin enthaltenen Inhaltsstoffe freizugeben. Der Film lässt die Keratinfasern, auf die er aufgetragen wird, füllig aussehen und "curlt" sie schön und dauerhaft. Zum gewünschten Zeitpunkt kann die Zubereitung leicht auch wieder entfernt werden, indem Wasser, ggf. mit Netzmittel, aufgebracht wird, was zu einem Quellen des Films führt, wodurch er leicht wieder abgenommen werden kann. Durch die spezielle Kombination von Wachskomponente. Filmbildner und Gelkomponente wird ein Netzwerk oder Gerüst gebildet, das dauerhaft an der Keratinfaser haftet, so dass ein homogener, voluminöser, stark haftender Film entsteht.

Ein weiterer Gegenstand der Erfindung ist ein Verfahren zur Erstellung dieser Zubereitung, wie in Anspruch 21 beschrieben.

Zur Herstellung der erfindungsgemäßen kosmetischen Zubereitung wird die wässrige Gelkomponente vorbereitet, indem das Hydrokolloid mit Wasser gequollen wird. Dazu wird bevorzugt erwärmtes Wasser, das vorteilhafterweise eine Temperatur zwischen 40°C und 100°C, bevorzugt ca. 65 bis 90°C hat, mit dem Hydrokolloid unter Rühren in Kontakt gebracht und anschließend danach ggf. abkühlen und quellen gelassen. Gegebenenfalls kann der Mischung noch ein Konservierungsmittel und/oder ein Dispergierhilfsmittel zugegeben werden. Das gequollene Gel ist stabil und kann auch über längere Zeiträume aufbewahrt werden, sodass es nicht sofort weiterverarbeitet werden muss.

Das für die Wachskomponente verwendete Wachs wird, gegebenenfalls zusammen mit dem Fett und/oder Öl aufgeschmolzen und bevorzugt auf eine Temperatur im Bereich zwischen 50 bis 100°C, bevorzugt 65 bis 85°C gebracht Die zur Herstellung der Emulsion notwendige Wassermenge, die Emulgatoren und Coemulgatoren enthalten kann, wird bevorzugt ebenfalls erwärmt und dann mit der Wachskomponente vermischt. Der entstehenden O/W-Emulsion werden dann das gequollene wässrige Hydrokolloid, gegebenenfalls das alkoholische Gel und die Dispersion des Filmbildners zugegeben. Das Vermischen erfolgt jeweils solange, bis eine homogene Dispersion bzw. Emulsion entstanden ist. Außerdem können dann gegebenenfalls die teilchenförmigen Bestandteile, insbesondere Pigmente und Füllstoffe, zugemischt werden, ggf. mit Hilfsstoffen, die die Kompatibilität verbessern.

Die Mischung wird dann gerührt bis sie homogen ist und dann unter Rühren abgekühlt. Gegebenenfalls können dieser Mischung entweder noch in warmem Zustand oder, falls es sich um empfindliche Zusatzstoffe handelt, während des Abkühlens die kosmetisch üblicherweise zugesetzten Wirkstoffe und Hilfsstoffe, wie Parfums, Weichmacher, Glanzstoffe etc. zugegeben werden.

Die erhaltene Zubereitung wird dann in Verpackungsbehälter abgefüllt. Da die erfindungsgemäße Zubereitung sich durch besondere Stabilität auszeichnet, kann sie auch in erwärmtem Zustand abgefüllt werden, wobei ein "Kaltrühren" nicht notwendig ist. Dies ist vorteilhaft einerseits aus hygienischen Gründen und andererseits, da in warmem Zustand die Fließfähigkeit in der Regel besser ist.

Für spezielle Ausführungsformen hat es sich als vorteilhaft erwiesen, einen Teil des Filmbildners der Wachskomponente zuzusetzen, was zu besonders ästhetischen Produkten führt. Insbesondere wird für diese Ausführungsform als Filmbildner für die Wachskomponente ein Vinylpyrrolidon-Copolymer verwendet.

Mit dem erfindungsgemäßen Verfahren kann die Zubereitung leicht, reproduzierbar und in hohen Stückzahlen hergestellt werden. Da die Zubereitung aufgrund ihrer Stabilität bei erhöhter Temperatur verarbeitet werden kann, ergeben sich sowohl in hygienischer als auch verfahrenstechnischer Hinsicht Vorteile gegenüber bekannten Produkten.

Die erfindungsgemäße Zubereitung ist besonders gut dazu geeignet, keratinische Materialien zu überziehen. Sie wird daher bevorzugt verwendet, um Wimpern, Augenbrauen, Haare, Haarteile, Barthaare und andere keratinische Bestandteile zu färben und/oder zu formen. Besonders geeignet ist die erfindungsgemäße Zubereitung als Mascara, da sie fest an Wimpern haftet, nicht ausblutet, nicht abbröckelt und lang und dauerhaft an der Wimper verbleibt, bis sie wieder entfernt wird.

Ein weiterer Gegenstand der Erfindung ist daher die Verwendung der erfindungsgemäßen Zubereitung zur Färbung und Formung von keratinischem Material und insbesondere zur Herstellung von Mascaras.

## Patentansprüche

1. Kosmetische Zubereitung in Form einer O/W-Emulsion, die neben üblichen kosmetischen Inhaltsstoffen eine Wachskomponente, ein Filmbildnersystem und mindestens eine Gelkomponente enthält, wobei die Gelkomponente mindestens ein gequollenes Hydrokolloid aufweist, wobei die kosmetische Zubereitung ein wässriges Gel und ein alkoholisches Gel enthält, wobei das alkoholische Gel ein mit einem einwertigen und/oder mehrwertigen Alkohol gequollenes Cellulosederivat ist, wobei der einwertige Alkohol insbesondere ein C₂-C₄-Alkohol ist und der mehrwertige Alkohol insbesondere ein C₂-C₆-Alkohol ist.

2. Kosmetische Zubereitung nach Anspruch 1, **dadurch gekennzeichnet, dass** die Wachskomponente ein oder mehrere Wachse enthält, die bei Raumtemperatur fest sind und einen Tropfpunkt von 50 bis 200°C haben.

3. Kosmetische Zubereitung nach einem der vorhergehenden Ansprüche, **dadurch gekennzeichnet, dass** in der Wachskomponente eine Kombination aus Emulgator und Coemulgator enthalten ist.

4. Kosmetische Zubereitung nach einem der vorhergehenden Ansprüche, **dadurch gekennzeichnet, dass** die Wachskomponente zusätzlich flüchtige Bestandteile, insbesondere synthetische Ester, Siliconöle, Dimethicone und Cyclomethicone und Isoparaffine in einer Konzentration von bis zu 20 % enthält.

5. Kosmetische Zubereitung nach einem der vorhergehenden Ansprüche, **dadurch gekennzeichnet, dass** das Filmbildnersystem ein filmbildendes Polymer ist, das in einem Medium dispergiert und/oder gelöst ist.

6. Kosmetische Zubereitung nach Anspruch 5, **dadurch gekennzeichnet, dass** das Filmbildnersystem ein anionisches, kationisches, nichtionisches und/oder amphoteres Polyurethan, Polyurethan-Acrylsäure-Polymer, Polyurethan-Polyvinylpyrrolidon, Polyester-Polyurethan, Polyether-Polyurethan, Polyharnstoff, Polyester, Polyesteramid, Polyester mit Fettkette, Polyamid, Epoxyesterharz, Acryl- und/oder Vinylpolymer, Acryl-Siliconcopolymer, Nitrocellulose/Acrylcopolymer, Polymer natürlichen Ursprungs, gegebenenfalls in modifizierter Form, Hybridpolymer, Polykondensationsprodukt, und/oder Silkoncopolymer oder eine Mischung von zwei oder mehr der vorher aufgezählten Polymere aufweist.

7. Kosmetische Zubereitung nach einem der vorhergehenden Ansprüche, **dadurch gekennzeichnet, dass** das Hydrokolloid ein wasserlösliches, natürliches oder synthetisches Polymer ist, das in Wasser quillt und ein Gel oder eine hochviskose Lösung bildet.

8. Kosmetische Zubereitung nach einem der vorhergehenden Ansprüche, **dadurch gekennzeichnet, dass** das Hydrokolloid ausgewählt ist aus Alginaten, Carageenen, Pektinen, Tragant, Gummen, Cellulosen und deren Derivaten, Stärken und Stärkederivaten, Polyvinylalkohol und Polyvinylpyrrolidon.

9. Kosmetische Zubereitung nach einem der vorhergehenden Ansprüche, **dadurch gekennzeichnet, dass** das wässrige Gel ein in Wasser gequollenes Stärkederivat ist.

10. Kosmetische Zubereitung nach einem der vorhergehenden Ansprüche, **dadurch gekennzeichnet, dass** das Cellulosederivat Hydroxymethylcellulose, Hydroxyethylcellulose, Hydroxypropylcellulose, Carboxymethylcellulose; Hydroxyalkylstärke oder Dimethylimidazolidinonstärke enthält.

11. Kosmetische Zubereitung nach einem der vorhergehenden Ansprüche, **dadurch gekennzeichnet, dass** das wässrige Gel zusätzlich mindestens ein Konservierungsmittel, und/oder mindestens ein Dispergierhilfsmittel und ggf. mindestens einen Weichmacher enthält.

12. Kosmetische Zubereitung nach Anspruch 10, **dadurch gekennzeichnet, dass** das gequollene Gel in einem Anteil von 3 bis 50 % enthalten ist.

13. Kosmetische Zubereitung nach einem der vorhergehenden Ansprüche, **dadurch gekennzeichnet, dass** die Wasseraufnahmekapazität des Hydrokolloids in einem Bereich von 100 bis 600 %, bevorzugt 200 bis 500 % liegt.

14. Kosmetische Zubereitung nach einem der vorhergehenden Ansprüche, **dadurch gekennzeichnet, dass** die Zubereitung zusätzlich eine Teilchenphase enthält.

15. Kosmetische Zubereitung nach einem der vorhergehenden Ansprüche, **dadurch gekennzeichnet, dass** die Teilchenphase aus anorganischen und/oder organischen Pigmenten, Füllstoffen und deren Gemischen besteht.

16. Kosmetische Zubereitung nach einem der vorhergehenden Ansprüche, **dadurch gekennzeichnet, dass** als Pigment Eisenoxide, Ultramarin, Chromoxidgrün, Chromoxidhydratgrün, Carbonblack, Titandioxid, Zinkoxid, Bariumsulfat, Carmin, Verlackungen organischer Färbemittel, insbesondere Aluminium-, Barium-, Calcium-, Kalium-, Strontium- und/oder Zinklacke, Talkum und/oder Kaolin enthalten sind.

17. Kosmetische Zubereitung nach einem der vorhergehenden Ansprüche, **dadurch gekennzeichnet, dass** als Füllstoffe Talkum, Kaolin, Bentonit, Hectorit, Montmorillonit, Ceroxid, Siliciumdioxid, Bornitrid, Nylonpulver, Polyethylenpulver, Polypropylenpulver, Seidenpulver, mehrwertige Metallseifen, nicht-quellbare Stärken, Fruchtfasern, Peelingkörper natürlichen und synthetischen Ursprungs, Sand, Kleieprodukte, Kernmehl von Steinfrüchten, Algenderivate, Duroplast-, Thermoplast- und/oder Elastomermehle und deren Gemische enthalten sind.

18. Kosmetische Zubereitung nach einem der vorhergehenden Ansprüche, **dadurch gekennzeichnet, dass** der Anteil der Teilchenphase bis zu 30 % beträgt.

19. Kosmetische Zubereitung nach einem der vorhergehenden Ansprüche, **dadurch gekennzeichnet, dass** die Wachskomponente in einem Anteil von 15 bis 30 %, das Filmbildnersystem in einem Anteil von 5 bis 60 %, die Gelkomponente in einem Anteil von 3 bis 50 %, jeweils bezogen auf das Gewicht der gesamten Zusammensetzung bezogen, enthalten sind, wobei der Rest Wasser und ggf. übliche Kosmetikhilfsstoffe sind.

20. Kosmetische Zubereitung nach einem der vorhergehenden Ansprüche, **dadurch gekennzeichnet, dass** als weitere Kosmetikhilfsstoffe, insbesondere Konservierungsmittel, Parfums, Extrakte, Proteinhydrolysat und/oder Seidenpulver enthalten sind.

21. Verfahren zur Herstellung einer gelhaltigen kosmetischen Zubereitung, wobei ein Hydrokolloid gegebenenfalls in Gegenwart eines Dispergierhilfsmittels und/oder eines Konservierungsmittels in Wasser gequollen wird; die Wachskomponente aufgeschmolzen wird, aus Wachskomponente, Wasser und ggf. Emulgatoren und Coemulgatoren eine Emulsion gebildet wird, der Emulsion das gequollene Hydrokolloid, Teilchenphase und Filmbildnersystem zugegeben werden und die Mischung gerührt wird, bis sie homogen ist, und ggf. kosmetisch übliche Inhaltsstoffe und Hilfsstoffe zugegeben werden, wobei der Mischung zusätzlich ein alkoholisches Gel aus mindestens einem einwertigen Alkohol und/oder mindestens einem mehrwertigen Alkohol und einem Gelbildner, insbesondere einem Cellulosederivat zugesetzt ist.

22. Verfahren nach Anspruch 21, **dadurch gekennzeichnet, dass** das Cellulosederivat vor der Zugabe gequollen wird.

23. Verfahren nach einem der Ansprüche 21 oder 22, **dadurch gekennzeichnet, dass** der pH-Wert der Mischung auf 6,5 bis 8,8 eingestellt wird.

24. Verwendung der kosmetischen Zubereitung nach einem der vorhergehenden Ansprüche zur Formung und/oder Färbung keratinischen Materials.

25. Verwendung einer Zubereitung nach einem der Ansprüche 1 bis 20 zur Herstellung eines Mascaras.

26. Verwendung nach Anspruch 24, **dadurch gekennzeichnet, dass** als keratinisches Material Augenwimpern, Augenbrauen, Kopfhaar, Haarteile und/oder Barthaar gefärbt und/oder geformt werden.

## Claims

1. A cosmetic preparation in the form of an oil-in-water emulsion which contains, in addition to standard cosmetic ingredients, a wax component, a film-forming system and at least one gel component, wherein the gel component has at least one swollen hydrocolloid, wherein the cosmetic preparation contains an aqueous gel and an alcoholic gel, wherein the alcoholic gel is a cellulose derivative that is swollen with a monohydric and/or polyhydric alcohol, wherein the monohydric alcohol is in particular a C₂-C₄ alcohol, and the polyhydric alcohol is in particular a C₂-C₆ alcohol.

2. A cosmetic preparation according to claim 1, **characterised in that** the wax component contains one or more waxes which are solid at room temperature and have a dropping point of 50 to 200°C.

3. A cosmetic preparation according to one of the preceding claims, **characterised in that** a combination of emulsifier and co-emulsifier is contained in the wax component.

4. A cosmetic preparation according to one of the preceding claims, **characterised in that** the wax component additionally contains volatile constituents, in particular synthetic esters, silicone oils, dimethicones and cyclomethicones and isoparaffins in a concentration of up to 20%.

5. A cosmetic preparation according to one of the preceding claims, **characterised in that** the film-forming system is a film-forming polymer which is dispersed and/or dissolved in a medium.

6. A cosmetic preparation according to claim 5, **characterised in that** the film-forming system has an anionic, cationic, non-ionic and/or amphoteric polyurethane, polyurethane acrylic-acid polymer, polyurethane polyvinyl pyrrolidone, polyester polyurethane, polyether polyurethane, polycarbamide, polyester, polyester amide, polyester with fat chain, polyamide, epoxy ester resin, acrylic and/or vinyl polymer, acrylic silicone copolymer, nitrocellulose/acrylic copolymer, polymer of natural origin, optionally in a modified form, hybrid polymer, polycondensation product, and/or silicone copolymer or a mixture of two or more of the polymers listed before.

7. A cosmetic preparation according to one of the preceding claims, **characterised in that** the hydrocolloid is a water-soluble, natural or synthetic polymer which swells in water and forms a gel or a highly viscous solution.

8. A cosmetic preparation according to one of the preceding claims, **characterised in that** the hydrocolloid is selected from alginates, carrageens, pectins, tragacanth, gums, cellulose and derivatives thereof, starches and starch derivatives, polyvinyl alcohol and polyvinyl pyrrolidone.

9. A cosmetic preparation according to one of the preceding claims, **characterised in that** the aqueous gel is a starch derivative that is swollen in water.

10. A cosmetic preparation according to one of the preceding claims, **characterised in that** the cellulose derivative contains hydroxymethyl cellulose, hydroxyethyl cellulose, hydroxypropyl cellulose, carboxymethyl cellulose, hydroxyalkyl starch or dimethylimidazolidinone starch.

11. A cosmetic preparation according to one of the preceding claims, **characterised in that** the aqueous gel additionally contains at least one preservative, and/or at least one dispersing aid and optionally at least one softening agent.

12. A cosmetic preparation according to claim 10, **characterised in that** the swollen gel is contained in a proportion of 3 to 50%.

13. A cosmetic preparation according to one of the preceding claims, **characterised in that** the water-absorbing capacity of the hydrocolloid lies in a range from 100 to 600%, preferably 200 to 500%.

14. A cosmetic preparation according to one of the preceding claims, **characterised in that** the preparation additionally contains a particle phase.

15. A cosmetic preparation according to one of the preceding claims, **characterised in that** the particle phase consists of inorganic and/or organic pigments, fillers and mixes thereof.

16. A cosmetic preparation according to one of the preceding claims, **characterised in that** iron oxides, ultramarine, chrome oxide green, hydrated chrome oxide green, carbon black, titanium dioxide, zinc oxide, barium sulphate, carmine, lakes produced from organic colourants, in particular aluminium, barium, calcium, potassium, strontium and/or zinc lakes, talcum and/or kaolin are contained as the pigments.

17. A cosmetic preparation according to one of the preceding claims, **characterised in that** talcum, kaolin, bentonite, hectorite, montmorillonite, cerium oxide, silicon dioxide, boron nitride, nylon powder, polyethylene powder, polypropylene powder, silk power, polyhydric metallic soaps, non-swelling starches, fruit fibres, peel bodies of natural and synthetic origin, sand, bran products, kernel meal of stone fruit, algae derivatives, duroplastic, thermoplastic and/or elastomer meal and mixes thereof are contained as the fillers.

18. A cosmetic preparation according to one of the preceding claims, **characterised in that** the proportion of the particle phase amounts to up to 30%.

19. A cosmetic preparation according to one of the preceding claims, **characterised in that** the wax component is contained in a proportion of 15 to 30%, the film-forming system is contained in a proportion of 5 to 60%, the gel component is contained in a proportion of 3 to 50%, in each case relative to the weight of the total composition, with the remainder being water and optionally standard auxiliary cosmetic materials.

20. A cosmetic preparation according to one of the preceding claims, **characterised in that** in particular preservatives, perfumes, extracts, protein hydrolysate and/or silk powder are contained as the further auxiliary cosmetic materials.

21. Method for producing a gel-containing cosmetic preparation, wherein a hydrocolloid is caused to swell in water optionally in the presence of a dispersing aid and/or a preservative; the wax component is melted down, an emulsion is formed from the wax component, water and optionally emulsifiers and co-emulsifiers, the swollen hydrocolloid, particle phase and film-forming system are added to the emulsion, and the mixture is stirred until it is homogeneous, and optionally cosmetically standard ingredients and auxiliary materials are added, wherein an alcoholic gel consisting of at least one monohydric alcohol and/or at least one polyhydric alcohol and a gelling agent, in particular a cellulose derivative, is/are additionally added to the mixture.

22. Method according to claim 21, **characterised in that** the cellulose derivative is caused to swell before the addition.

23. Method according to one of claims 21 or 22, **characterised in that** the pH value of the mixture is adjusted to 6.5 to 8.8.

24. Use of the cosmetic preparation according to one of the preceding claims for the shaping and/or colouring of keratin material.

25. Use of a preparation according to one of claims 1 to 20 for the production of a mascara.

26. Use according to claim 24, **characterised in that** as the keratin material eyelashes, eyebrows, head hair, hair-pieces and/or beard hair are coloured and/or shaped.

## Revendications

1. Préparation cosmétique sous la forme d'une émulsion huile-dans-eau, qui contient, en plus des ingrédients cosmétiques usuels, un constituant cireux, un système filmogène et au moins un constituant gélifiant, dans laquelle le constituant gélifiant présente au moins un hydrocolloïde gonflé, dans laquelle la préparation cosmétique contient un gel aqueux et un gel alcoolique, dans laquelle le gel alcoolique est un dérivé de cellulose gonflé avec un alcool monovalent et/ou polyvalent, où l'alcool monovalent est en particulier un alcool en C₂ à C₄ et l'alcool polyvalent est en particulier en alcool en C₂ à C₆.

2. Préparation cosmétique selon la revendication 1, **caractérisée en ce que** le constituant cireux contient une ou plusieurs cires, qui sont solides à la température ambiante et ont une température de suintement comprise de 50 à 200 °C.

3. Préparation cosmétique selon l'une des revendications précédentes, **caractérisée en ce que** le constituant cireux contient une combinaison d'un émulsifiant et d'un coémulsifiant.

4. Préparation cosmétique selon l'une des revendications précédentes, **caractérisée en ce que** le constituant cireux contient en outre des constituants volatiles, en particulier des esters synthétiques, des huiles de silicone, des diméthicones, des cyclométhicones et de l'isoparaffine, selon une concentration allant jusqu'à 20 %.

5. Préparation cosmétique selon l'une des revendications précédentes, **caractérisée en ce que** le système filmogène est un polymère filmogène qui est dispersé et/ou dissous dans un milieu.

6. Préparation cosmétique selon la revendication 5, **caractérisée en ce que** le système filmogène présente un polyuréthane anionique, cationique, non ionique et/ou amphotère, un polymère de polyuréthane-acide acrylique, un polyuréthane-polyvinylpyrrolidone, un polyester-polyuréthane, un polyéther-polyuréthane, une polyurée, un polyester, un polyesteramide, un polyester avec une chaîne grasse, un polyamide, une résine d'époxy-ester, un polymère d'acryle et/ou de vinyle, un copolymère d'acryle-silicone, un copolymère de nitrocellulose/acryle, un polymère d'origine naturelle, éventuellement sous une forme modifiée, un polymère hybride, un produit de polycondensation et/ou un copolymère de silicone, ou un mélange de deux des polymères précédemment cités ou plus.

7. Préparation cosmétique selon l'une des revendications précédentes, **caractérisée en ce que** l'hydrocolloïde est un polymère hydrosoluble, naturel ou synthétique, qui gonfle dans l'eau et forme un gel ou une solution très visqueuse.

8. Préparation cosmétique selon l'une des revendications précédentes, **caractérisée en ce que** l'hydrocolloïde est sélectionné parmi des alginates, du carraghénane, des pectines, de la gomme adragante, des gommes, des celluloses et ses dérivés, des amidons et des dérivés d'amidon, de l'alcool de polyvinyle et de la polyvinylpyrrolidone.

9. Préparation cosmétique selon l'une des revendications précédentes, **caractérisée en ce que** le gel aqueux est un dérivé d'amidon gonflé dans de l'eau.

10. Préparation cosmétique selon l'une des revendications précédentes, **caractérisée en ce que** le dérivé de cellulose contient de l'hydroxyméthylcellulose, de l'hydroxyéthylcellulose, de l'hydroxypropylcellulose, de la carboxyméthylcellulose, de l'hydroxyalkylamidon ou du diméthylimidazolidinone-amidon.

11. Préparation cosmétique selon l'une des revendications précédentes, **caractérisée en ce que** le gel aqueux contient en outre au moins un agent conservateur, et/ou au moins un auxiliaire de dispersion et éventuellement au moins un plastifiant.

12. Préparation cosmétique selon la revendication 10, **caractérisée en ce que** le gel gonflé est présent selon une proportion comprise entre 3 et 50 %.

13. Préparation cosmétique selon l'une des revendications précédentes, **caractérisée en ce que** la capacité d'absorption d'eau de l'hydrocolloïde est comprise dans une plage allant de 100 à 600 %, de préférence de 200 à 500 %.

14. Préparation cosmétique selon l'une des revendications précédentes, **caractérisée en ce que** la préparation contient en outre une phase particulaire.

15. Préparation cosmétique selon l'une des revendications précédentes, **caractérisée en ce que** la phase particulaire se compose de pigments inorganiques et/ou organiques, de matières de charge et de leurs mélanges.

16. Préparation cosmétique selon l'une des revendications précédentes, **caractérisée en ce que** sont présents en tant que pigment des oxydes de fer, du bleu d'outremer, du vert d'oxyde de chrome, du vert d'hydrate de chrome, du noir de carbone, du dioxyde de titane, de l'oxyde de zinc, du sulfate de baryum, du carmin, des formations de pigments à base de colorants organiques, en particulier des vernis d'aluminium, de baryum, de calcium, de potassium, de strontium et/ou de zinc, du talc et/ou du kaolin.

17. Préparation cosmétique selon l'une des revendications précédentes, **caractérisée en ce que** sont présents en tant que matières de charge du talc, du kaolin, de la bentonite, de l'hectorite, de la montmorillonite, de l'oxyde de cérium, du dioxyde de silicium, du nitrure de bore, de la poudre de nylon, de la poudre de polyéthylène, de la poudre de polypropylène, de la poudre de soie, des savons métalliques polyvalents, des amidons non gonflables, des fibres extraites de fruits, des agents exfoliants d'origine naturelle et synthétique, du sable, des produits du son, de la fleur de farine issue de fruits à noyaux, des dérivés d'algues, des farines duroplastes, thermoplastes et/ou élastomères, et leurs mélanges.

18. Préparation cosmétique selon l'une des revendications précédentes, **caractérisée en ce que** la fraction de la phase particulaire va jusqu'à 30 %.

19. Préparation cosmétique selon l'une des revendications précédentes, **caractérisée en ce que** le constituant cireux est présent selon une proportion comprise entre 15 et 30 %, le système filmogène est présent selon une proportion comprise entre 5 et 60 %, le constituant gélifiant est présent selon une proportion comprise entre 3 et 50 %, respectivement sur la base du poids de l'ensemble de la composition, le reste étant de l'eau et éventuellement des excipients cosmétiques usuels.

20. Préparation cosmétique selon l'une des revendications précédentes, **caractérisée en ce qu'**elle contient comme autres excipients cosmétiques en particulier des conservateurs, des parfums, des extraits, un hydrolysat de protéine et/ou de la poudre de soie.

21. Procédé de fabrication d'une préparation cosmétique contenant un gel, dans lequel un hydrocolloïde est gonflé dans de l'eau, éventuellement en présence d'un auxiliaire de dispersion et/ou d'un agent de conservation ; le constituant cireux est fondu, une émulsion est formée à partir du constituant cireux, de l'eau et éventuellement des émulsifiants et coémulsifiants ; on ajoute à l'émulsion l'hydrocolloïde gonflé, la phase particulaire et le système filmogène, puis le mélange est agité jusqu'à ce qu'il soit homogène, et on ajoute éventuellement des constituants et excipients usuels sur le plan cosmétique, suite à quoi on ajoute en outre au mélange un gel alcoolique composé d'au moins un alcool monovalent et/ou d'au moins un alcool polyvalent et d'un agent filmogène, en particulier d'un dérivé de cellulose.

22. Procédé selon la revendication 21, **caractérisé en ce que** le dérivé de cellulose est gonflé avant l'ajout.

23. Procédé selon l'une des revendications 21 ou 22, **caractérisé en ce que** la valeur de pH du mélange est réglée entre 6,5 et 8,8.

24. Utilisation d'une préparation cosmétique selon l'une des revendications précédentes pour mettre en forme et/ou colorer des matériaux kératineux.

25. Utilisation d'une préparation selon l'une des revendications 1 à 20 pour fabriquer un mascara.

26. Utilisation selon la revendication 24, **caractérisée en ce que** l'on colore et/ou met en forme en tant que matériau kératineux des cils, des sourcils, des cheveux, des mèches de cheveux et/ou des poils de barbe.
